# EUROPEAN PATENT APPLICATION

(11) **EP 4 781 831 A1**
(43) Date of publication of application: **29.07.2026**
(21) Application number: 25472002.2
(22) Date of filing: 22.01.2025
(51) Int. Cl.: A23B 2/733, A23B 4/20, A61K 36/53

(54) **NATURAL COMPOSITION OF BIOPRESERVATIVE**

(71) Applicant: "Bioconserv" OOD, 3060 Krivodol (BG)
(72) Inventor: Titorenkov, Vladimir Aleksandrov, 1000 Sofia (BG); Nikolova, Milena Tihomirova, 1320 Bankia (BG); Berkov, Strahil, 2050 Ihtiman (BG); Nikolova, Tihomirova Milena, 1000 Sofia (BG)
(74) Representative: Benatov, Samuil Gabriel

(57) **Abstract**

The present invention relates to a natural composition of a biopreservative, which will find application in the food industry, in particular it will serve as a means of influencing by suppressing the growth of several types of microorganisms, which are the main cause of rapid spoilage of foods and especially soft drinks, with natural ingredients. The natural composition of the biopreservative consists of a phenolic fraction extracted from a mixture of dry and/or fresh plants. The said mixture includes rosemary (Rosmarinus officinalis) from 5 to 40 wt.% and thyme (Thymus Vulgaris) from 5 to 50 wt. % and sage (Salvia officinalis) from 5 to 40 wt.%. The said phenolic fraction contains from 8 to 98 wt.% phenolic acids relative to the total content of the composition.

## Description

### Field of technology

The present invention relates to a natural composition of a biopreservative, which will find application in the food industry, in particular it will serve as a means of suppressing the growth of certain microorganisms, which are the main cause of rapid spoilage of foods and especially non-alcoholic beverages, with natural ingredients.

### Prior art

Various methods and means for inhibiting the growth of microorganisms in food products are known from practice, of which the main categories are physical methods, biological methods, chemical agents, among others.

Physical methods are popular for food preservation and most often include heat treatment such as pasteurization and sterilization, which kill pathogens and reduce the number of spoilage microorganisms by moderate heating, or complete destruction of microorganisms by high-temperature treatment. Other types of physical methods include freezing, which inhibits the growth of microorganisms but does not completely destroy them, or drying, which reduces the available water (water activity) necessary for the growth of microorganisms, as well as irradiation, which uses ionizing radiation (gamma rays or electron beams) to destroy pathogens. Physical methods for inhibiting the growth of microorganisms are widely used, but have an impact on the quality of food products, such as loss of nutrients (e.g. vitamins) during heat treatment, changes in the texture, taste and color of foods (especially during heat treatment, freezing, drying and irradiation), and damage to the cellular structure of foods (e.g. freezing).

Biological methods for suppressing microorganisms are an innovative approach that uses natural interactions between organisms or compounds produced by them to limit food spoilage. They include the use of biopreservatives such as bacteriocins, probiotics and fermentation technologies. The main idea of these methods is to create an environment that is unfavorable for the development of pathogenic and spoilage microorganisms, while maintaining the quality and safety of food products. Despite their many advantages, biological methods also have their drawbacks. They are often highly dependent on specific storage conditions, such as temperature, humidity and pH, which must be precisely controlled to achieve effectiveness. The instability of some biological agents when these conditions change can lead to loss of activity, which limits their application in various foods. In addition, in some cases, interaction between biopreservatives and food ingredients is possible, which can affect their effect. The production of biological preservatives is relatively expensive and requires specialized technology, which increases the cost of the final product. Disadvantages of biological methods also include the risk of developing resistance in microorganisms if these technologies are not used correctly. For example, the prolonged use of bacteriocins can lead to pathogen adaptation and a decrease in the effectiveness of the method. In addition, consumers may be skeptical of products with added microorganisms, especially if there is a lack of sufficient information about their safety.

Chemical agents to inhibit microorganisms are among the most widely used methods of food preservation. They involve the addition of synthetic or natural compounds that inhibit the growth of pathogenic and spoilage microorganisms. The most commonly used chemical agents are organic acids, nitrates and nitrites, sulfites, various types of antimicrobial enzymes, and phenolic compounds, such as the widely used sodium benzoate. These compounds act by creating unfavorable conditions for growth, such as lowering pH, inhibiting the enzyme systems of microorganisms, or directly destroying their cell structure. Chemical agents offer significant advantages, including ease of application, long-lasting effects, and the ability to be used on a variety of foods. They are often cost-effective and easy to implement on an industrial scale. In addition, some of them, such as salts and sugars, are traditionally used in the food industry, which makes them more likely to be accepted by consumers. In particular, the aforementioned sodium benzoate is considered safe, but it has been found that negative side effects occur when it is mixed with ascorbic acid (vitamin C, a common gradient in drinks) and benzene is produced, which is carcinogenic.

### Summary of the invention

The objective of the present invention is to create an improved alternative chemical agent in the form of a biopreservative, which has a natural origin and is extracted from plants.

The problem is solved by a natural composition of a biopreservative, which consists of a phenolic fraction extracted from a mixture of dry and/or fresh plants. Said mixture includes rosemary (Rosmarinus officinalis) from 5 to 40 wt.%, thyme (Thymus Vulgaris) from 5 to 50 wt.% and sage (Salvia officinalis) from 5 to 40 wt.%. Said phenolic fraction contains from 8 to 98 wt.% phenolic acids relative to the total content of the composition.

In one embodiment, according to the present invention, said mixture of dry and/or fresh plants may additionally include white willow (Salix alba) from 20 to 60 wt.% and/or nettle (Urtica dioica) from 5 to 35 wt.% and/or oregano (Origanum vulgare) from 5 to 35 wt.% and/or mint (Menta piperita) from 3 to 10 wt.%.

In embodiments according to the present invention, the phenolic fraction may be in the form of a powdered concentrate or a purified liquid extract.

The natural composition of the biopreservative is extracted from plants, therefore it is a natural product. The composition affects a wide range of identified microorganisms, as well as the biological systems of some fungi and molds in a way created by natural inter-organism relationships. The ingredients have a number of proven characteristics beneficial to human health, as they do not cause accumulation and at the same time suppress the development of microorganisms without killing them, thus extending the shelf life of food products. In combination of several extracts, a synergistic effect is achieved, expressed in strengthening and expanding the spectrum of action of the biopreservative.

### Exemplary embodiments of the invention

The natural composition of the biopreservative according to the present invention consists of a phenolic fraction extracted from a mixture of dry and/or fresh plants. Said mixture of dry and/or fresh plants is based on a combination of rosemary (Rosmarinus officinalis), thyme (Thymus Vulgaris) and sage (Salvia officinalis), where the content of the individual components in the composition is in the following amounts (in wt.%):
- rosemary can be present in an amount of 5 to 40 wt.%, relative to the total mass of the plant raw material, and preferably, for example, it can be: 5, 20, 27 and 40 wt.%;
- the thyme may be present in an amount of 5 to 50 wt. %, relative to the total mass of the plant raw material, and preferably may be, for example: 5, 10, 30, 33 or 50 wt. %;
- the sage can be in an amount of 5 to 40 wt.%, relative to the total mass of the plant raw material, and preferably it can be, for example: 5, 15, 30 or 40 wt.%.

In alternative embodiments, said mixture of dry and/or fresh plants may additionally include one or a combination of the following white willow (Salix alba) and/or stinging nettle (Urtica dioica) and/or oregano (Origanum vulgare) and/or mint (Menta piperita), where the content of the individual components in the composition is in the following amounts (in wt.%):
- white willow can be present in an amount of 20 to 60% by weight, relative to the total mass of the plant raw material;
- nettle can be present in an amount of 5 to 35 wt.%, relative to the total mass of the plant raw material;
- oregano can be present in an amount of 5 to 35 wt.%, relative to the total mass of the plant raw material.
- the mint can be in an amount of 3 to 10 wt.%, relative to the total mass of the plant raw material, and preferably, for example, it can be 5 wt.%.

The natural composition of a biopreservative, according to the present invention, is presented with the following specific exemplary embodiments, which, through the unique characteristics of the individual ingredients, achieve the desired effects and which disclose combinations of the features of the description, falling within the scope of the present invention, as defined by the claims, where:
Example 1 - natural composition of biopreservative based on rosemary (Rosmarinus officinalis) and thyme (Thymus Vulgaris) and sage (Salvia officinalis), containing:
   - rosemary - 27 wt. %;
   - thyme - 33 wt. %;
   - sage - 40 wt. %.
Example 2 - natural composition of biopreservative based on rosemary (Rosmarinus officinalis) and thyme (Thymus Vulgaris), sage (Salvia officinalis) and white willow (Salix alba), containing:
   - rosemary - 20 wt. %;
   - thyme - 30 wt. %;
   - sage - 30 wt. %;
   - white willow - 20 wt. %.
Example 3 - natural composition of biopreservative based on rosemary (Rosmarinus officinalis) and thyme (Thymus Vulgaris), sage (Salvia officinalis), white willow (Salix alba), nettle (Urtica dioica) and oregano (Óriganum vulgare) containing:
   - rosemary - 5 wt. %;
   - thyme - 10 wt. %;
   - sage - 15 wt. %;
   - white willow - 60 wt. %;
   - nettle - 5 wt. %;
   - oregano - 5 wt. %.
Example 4 - natural composition of biopreservative based on rosemary (Rosmarinus officinalis), thyme (Thymus Vulgaris), sage (Salvia officinalis), white willow (Salix alba), nettle (Urtica dioica) and peppermint (Menta piperita) containing:
   - rosemary - 5 wt. %;
   - thyme - 10 wt. %;
   - sage - 15 wt. %;
   - white willow - 60 wt. %;
   - nettle - 5 wt. %;
   - mint - 5 wt. %.

In embodiments according to the present invention, the phenolic fraction may be in the form of a purified liquid extract or a powder concentrate.

The purified liquid extract of the phenolic fraction is obtained by aqueous extraction of the plant mixture of dry and/or fresh plants, initially significantly reducing the characteristic plant aromas by aqueous distillation of the aromatic components in a specialized distiller. Then, repeated extraction is performed with 30° ÷ 90° ethyl alcohol for a period of 24 ÷ 56 hours at room temperature or for a shorter time under heating and high pressure. The mechanical residues from the plant mixture are then separated by mechanical cascade filtration. The ethyl alcohol is distilled until it separates into two phases - a light aqueous phase is separated from a heavy dark green phase containing chlorophyll. Subsequently, chlorophyll is removed from the intermediate product (or product) by precipitation and filtration, resulting in a purified liquid extract enriched with phenolic substances.

The powdered concentrate of the phenolic fraction is obtained by alkaline hydrolysis of the mixture of dry and/or fresh plants with 0.5N ÷ 2N NaOH for 24 ÷ 56 h at room temperature or upon heating, after which the free and hydrolyzed phenolic acids are extracted with ethyl acetate by classical liquid-liquid extraction or by liquid-liquid extraction using a diatomaceous earth column after acidification to pH 1 ÷ 4. This is followed by elimination of the ethyl acetate by distillation or spray drying.

A powdered concentrate of the phenolic fraction can also be obtained by alkaline hydrolysis of the mixture of dry and/or fresh plants with 0.5N ÷ 2N NaOH for 24 ÷ 56 h at room temperature or under heating, after which the resulting extract containing free and hydrolyzed phenolic acids is loaded after acidification to pH 1 ÷ 4 into a column with ion exchange resins. Neutral substances are washed with water and the phenolic fraction is extracted with a water-ethanol solution (30-90 vol. % ethanol).

A powdered concentrate of the phenolic fraction can also be obtained from the purified liquid extract by eliminating the solvents.

The solvents are eliminated by lyophilization or spray drying to obtain a powder concentrate of the phenolic fraction containing from 8 to 75 wt.% phenolic acids, determined by the Folin spectrophotometric method. The product has high water solubility and is packaged in powder form. According to the present invention, said phenolic fraction contains from 8 to 98 wt.% phenolic acids relative to the total content of the composition, which was established by performing experiments on the composition of the biopreservative of examples 1-4, in which the content of phenolic acids was determined by gas chromatography-mass spectrometry.

**Table 1 - Phenolic fraction from example 1 in the form of purified liquid extract and powder concentrate, where the values represent % of the total ion flux by the gas chromatography-mass spectrometry method.**

| Phenolic fraction | Purified extract | Powder concentrate |
|---|---|---|
| 3-Hydroxybenzoic acid | 4.55 | 12.44 |
| Quinic acid | 0.84 | 3.66 |
| Salicylic acid | 0.01 | 0.19 |
| Protocatechuic acid | 0.12 | 1.18 |
| Vanillic acid | 0.36 | 2.99 |
| m-Coumaric acid | 0.12 | 0.33 |
| Catechol lactate | 1.00 | 5.45 |
| Caffeic acid | 0.37 | 4.28 |
| Ferulic acid | 1.61 | 15.50 |
| Caffeic acid deriv. | 0.14 | 3.18 |
| Rosmarinic acid | 1.12 | 7.81 |
| Sum of phenolic substances | 9.11 | 57.64 |

Additionally, experiments were conducted in which the contents of phenolic acids in the individual main ingredients - rosemary, thyme and sage, were determined by gas chromatography-mass spectrometry, as shown in Tables 2, 3 and 4.

**Table 2 - Phenolic fraction of thyme in the form of purified liquid extract and powder concentrate, where the values represent % of the total ion flux by the gas chromatography-mass spectrometry method.**

| Phenolic fraction | Purified extract | Powder concentrate |
|---|---|---|
| 3-Hydroxybenzoic acid | 0.09 | 4.39 |
| Unidentified phenolic acid | - | 1.73 |
| Vanillic acid | - | 1.35 |
| Unidentified phenolic acid | - | 0.83 |
| Protocatechuic acid | 0.74 | 28.05 |
| Coumaric acid | - | 1.94 |
| Syringic acid | - | 0.35 |
| Quinic acid | 7.04 | - |
| Caffeic acid | 0.04 | - |
| Unidentified phenolic acid | - | 13.44 |
| 3,4-Dihydroxyphenylacetic acid | 0.1 | - |
| Catechol lactate | 5.53 | 24.35 |
| Ferulic acid | - | 0.38 |
| Caffeic acid trans | 1.08 | 24.06 |
| Genistic acid | - | 0.51 |
| Rosmarinic acid | 0.01 | 0.18 |
| Sum of phenolic substances | 14.63 | >98 |

**Table 3 - Phenolic fraction of rosemary in the form of purified liquid extract and powder concentrate, where the values represent % of the total ion flux by the gas chromatography-mass spectrometry method.**

| Phenolic fraction | Purified extract | Powder concentrate |
|---|---|---|
| Salicylic acid | 0.1 | 0.8 |
| 3-Hydroxybenzoic acid | 0.1 | 0.6 |
| Vanillic acid | 0.1 | 0.6 |
| Protocatechuic acid | 2.21 | 16.21 |
| 3,4-Dihydroxyphenylacetic acid | 0.1 | 0.9 |
| Quinic acid | 6.72 | 16.72 |
| p-Hydroxycinnamic acid | 0.1 | 0.8 |
| Catechol lactate | 3.95 | 13.05 |
| Ferulic acid | 0.13 | 1.02 |
| Caffeic acid | 3.15 | 16.19 |
| Rosmarinic acid | 1.33 | 11.01 |
| Sum of phenolic substances | 17.99 | 77.9 |

**Table 4 - Phenolic fraction of sage in the form of purified liquid extract and powder concentrate, where the values represent % of the total ion flux by the gas chromatography-mass spectrometry method.**

| Phenolic fraction | Purified extract | Powder concentrate |
|---|---|---|
| Salicylic acid | 0.12 | 0.89 |
| 1-Hydroxy-2-methoxy-benzene | - | 0.18 |
| p-Hydroxycinnamic acid | 0.1 | 2.55 |
| Catechol lactate | 1.3 | 3.86 |
| Ferulic acid | 0.1 | 1.66 |
| Caffeic acid | 0.55 | 11.08 |
| Vanillic acid | - | 0.1 |
| 4-Hydroxy-3-methoxyacetophenone | - | 0.1 |
| Sum of phenolic substances | 2.17 | 20.42 |

Similarly, based on the properties of white willow, nettle, oregano and mint, it has been found that these plants can complement the properties of the biopreservative, providing additional antioxidant and antimicrobial activity. The data obtained show that the addition of combinations of phenolic fractions from the listed plants not only enriches the composition, but also expands the applicability of the biopreservative in various industrial sectors.

The natural composition of the biopreservative, according to the present invention, serves as a natural antimicrobial agent in the food industry, as it exhibits significant antimicrobial activity due to the substances contained in the phenolic fraction, which is extracted from the mixture of dry and/or fresh plants and which is rich in phenolic acids. The natural composition of the biopreservative acts primarily at the level of the cell membrane of microorganisms, which leads to the compromise of cell integrity and inhibition of vital processes such as respiration and transport of substances. In addition, the biopreservative can interact with enzymes in microorganisms, blocking their functions and preventing growth. These properties make it effective against a wide range of pathogenic and spoilage microorganisms, including bacteria, yeasts and molds. Another important aspect is that phenolic acids are natural compounds that are part of the human diet, which makes the natural composition of the biopreservative attractive from the point of view of safety and consumer perception. Its use as a preservative reduces the need for synthetic chemical agents, which is in line with the trend towards cleaner labels and the preference for natural ingredients. In addition to its antimicrobial properties, the natural composition of the biopreservative also has antioxidant activity, which further contributes to the preservation of foods by preventing oxidative processes that can lead to quality deterioration. The natural composition of the biopreservative is also compatible with different food matrices and can be used in a wide variety of products, such as meat, dairy products, beverages and baked goods. It not only extends the shelf life , but can also improve the nutritional profile of products by adding bioactive compounds with potential health benefits. All this shows that the natural composition of the biopreservative is a promising natural preservative that combines antimicrobial and antioxidant activity with safety and compatibility with food products. Its effectiveness and naturalness make it a valuable product in the modern food industry, especially in the context of increasing demands for sustainability and healthy foods.

Although the present invention has been described in connection with preferred embodiments, it should be noted that other variants of embodiments are possible, obvious to a person skilled in the art, which will achieve the desired effects, by the features disclosed in the present description and claims, which do not depart from the scope of the invention as defined by the claims.

## Claims

1. Natural composition of biopreservative, **characterized in that** it consists of a phenolic fraction extracted from a mixture of dry and/or fresh plants, which mixture includes rosemary (*Rosmarinus officinalis*) from 5 to 40 wt.%, thyme (*Thymus Vulgaris*) from 5 to 50 wt.% and sage (*Salvia officinalis*) from 5 to 40 wt.%, wherein said phenolic fraction contains from 8 to 98 wt.% phenolic acids relative to the total content of the composition.

2. Natural composition of biopreservative according to claim 1, **characterized in that** said mixture of dry and/or fresh plants additionally includes white willow (*Salix alba*) from 20 to 60 wt.% and/or nettle (*Urtica dioica*) from 5 to 35 wt.% and/or oregano (*Origanum vulgare*) from 5 to 35 wt.% and/or mint (*Menta piperita*) from 3 to 10 wt.%.

3. Natural composition of biopreservative according to claim 1 or 2, **characterized in that** said phenolic fraction is a powdered concentrate or a purified liquid extract.
